# EUROPEAN PATENT APPLICATION

(11) **EP 1 709 977 A1**
(43) Date of publication of application: **11.10.2006**
(21) Application number: 05007251.1
(22) Date of filing: 02.04.2005
(51) Int. Cl.: A61L 2/07, A61L 2/02

(54) **Method for sterilization of equipment used at cryogenic temperatures**

(71) Applicant: Linde Aktiengesellschaft, 65189 Wiesbaden (DE)
(72) Inventor: Ekeblad, Per Olof, 184 92 Akersberga (SE); Johansson, Sven-Ake, 125 41 Alvsjo (SE); Nordström, Marika, 169 71 Solna (SE)
(74) Representative: Gellner, Bernd

(57) **Abstract**

The invention relates to a method for sterilizing in place of equipment (9, 11,17, 4,12) wherein in operation said equipment (9, 11,17, 4,12) is used at cryogenic temperatures. The method comprises the steps of passing steam through said equipment (9, 11,17, 4,12) and drying said equipment (9, 11,17, 4,12) by introducing a gas into said equipment (9, 11,17, 4,12). Prior to passing steam through said equipment (9, 11,17, 4,12), said equipment (9, 11,17, 4,12) is pre-heated by introduction of a gas. (Figure 2)

## Description

The invention relates to a method for sterilizing in place of equipment wherein in operation said equipment is used at cryogenic temperatures comprising the steps of passing steam through said equipment and drying said equipment by introducing a gas into said equipment.

The production of pharmaceuticals in industrial scale is made in fully automated plants which have to fulfil strict authority regulations. When in the production of pharmaceuticals cryogenic liquids are used which are in direct contact with the pharmaceutical, sterile cryogenic liquids are required.

To produce a sterile cryogenic liquid a commercially available sterile filter may be used. These filters are normally made to handle liquids and gases at approximate ambient temperatures. Using them at cryogenic temperatures and with cryogenic liquids involves a number of problems. One of them is that such a filter generates a pressure drop which will cause a non sub cooled cryogenic liquid to flash. The pressure wave resulting from the flashing liquid might in a non-determinable way deteriorate the filter.

EP 0 872 250 B1 suggests a method for producing sterile liquid nitrogen wherein non sterile liquid nitrogen is vaporized in an evaporator and passed through a biological filter in order to produce a sterile vapour. The sterile vapour is cooled in one heat exchanger and is then finally liquified in a condenser and resulting sterile liquid nitrogen is withdrawn.

From time to time it is necessary to sterilize the whole equipment which is used in the production of the sterile cryogenic liquid. Sterilization is normally carried out by passing steam through the equipment to be sterilized. Sterilization in place of equipment which during normal operation contains liquified gases includes the risk that residues of water remain in the equipment and cause problems or failure when the equipment is cooled down. Further, residues of water may contaminate the sterile cryogenic liquid as the specified limits of how much water the product may contain are often very narrow.

In EP 0 872 250 B1 prior to the start of the sterile nitrogen production steam is passed through the biological filter for several hours to sterilize the filters and all attendant passages. The steam is then turned off and gas is passed through the equipment in order to dry it. However, by that method it is not possible to sterilize the equipment after the evaporator once has been cooled down since any steam introduced into the equipment would freeze and clog the evaporator.

Thus it is an object of the invention to provide an improved method for sterilizing in place of equipment which in normal operation contains liquified gases.

This object is achieved by a method for sterilizing in place of equipment wherein in operation said equipment is used at cryogenic temperatures comprising the steps of passing steam through said equipment and drying said equipment by introducing a gas into said equipment, wherein prior to passing steam through said equipment, said equipment is pre-heated by introduction of a gas.

The term "sterilization in place" means that the equipment is not taken apart for sterilization, but is left in the same arrangement as in operation.

For example in the production of sterile cryogenic liquids, it is necessary to sterilize the equipment from time to time in order to keep the quality requirements of the sterile product. The invention provides a method which enables a quick sterilization in place of equipment which during normal operation contains liquified gases. Thus it is possible to interrupt the normal production process for sterilization of the equipment and after sterilization has taken place to continue with the production process. The inventive method can be fully automated, but can also be used in semi automatic or manual mode.

According to the invention the equipment which has been used at cryogenic temperatures is first pre-heated by introduction of a gas. The pre-heating step is carried out until a pre-determined temperature within the equipment has been reached. Preferably the temperature of the gas leaving the equipment is measured and used as an indicator whether or not the pre-heating shall be continued. It is also possible to have one or more temperature sensors located at certain places within the equipment.

As already mentioned the pre-heating step is performed until a pre-defined temperature has been reached. That pre-defined temperature is preferably between 5°C and 30°C, more preferred between 10°C and 20°C. In any case the pre-defined temperature should be above 0°C in order to avoid freezing when steam enters the equipment.

When the pre-heating step has finished clean steam is passed through the equipment for sterilization. Preferably said clean steam is introduced into the equipment until all parts of the equipment have reached the sterilization temperature which is preferably between 110°C and 130 °C.

In order to ensure that all parts of the equipment are sterilized it is advantageous to keep the temperature within the equipment at or above the sterilization temperature for at least 15 minutes. That means the flow of clean steam is continued for that period of time.

Further the geometrical design of said equipment is preferably made in such a way that all parts of said equipment are reached by said steam.

After sterilization has taken place it is necessary to dry the equipment before it is cooled down to cryogenic temperatures. This is done by introducing a dry gas into all parts of the equipment. Preferably the dry gas is heated prior to entering the equipment. The heating of the dry gas may be carried out by any suitable means for heating, but it has proven advantageous to heat the dry gas in indirect heat exchange with steam. The heating of the dry gas may be carried out by indirect heat exchange with the clean steam used for sterilization or by any other steam or hot medium. Since the hot medium does not come into contact with the sterile parts of the equipment there are no strict requirements as to the purity of the hot medium and it is for example possible to use simple plant steam for that purpose.

It is advantageous to pre-heat and / or dry the equipment by introduction of a gas of the same type as used in normal operation. In practice normally one of nitrogen, oxygen, carbon dioxide, argon, or any mixtures of these gases is used.

During the sterilization step some steam condenses and water may collect in some parts of the equipment. Thus, in order to speed up the subsequent drying step it is advantageous to design the whole equipment in a way that all parts to be sterilized are completely drainable.

During normal operation of the equipment at cryogenic temperatures it has to be ensured that no steam enters the equipment. Otherwise water may freeze and clog parts of the equipment. Hence, according to a preferred embodiment of the invention, said steam is introduced into said equipment via a steam inlet line comprising at least one valve and a condensate drain line branching upstream said valve wherein said condensate drain line is provided with a shut off valve.

The condensate drain line with said shut off valve functions as a steam trap. The shut off valve comprises a capsule containing a special liquid with a boiling point below that of water. In cold conditions the capsule is relaxed and the shut off valve is open. During the sterilization step the valve in said steam inlet line is open and hot steam is allowed to enter the equipment. As hot condensate passes through the condensate drain line to the shut off valve, heat is transferred to the liquid in the capsule. The liquid in the capsule boils before steam reaches the shut off valve. The increasing vapour pressure causes the capsule to expand and the shut off valve closes. Thus all steam will flow into the equipment.

During normal operation the valve in said steam inlet line is closed. In case of a leakage in said valve the steam can be drained through the condensate drain line since the shut off valve which is at low temperature is open.

It is advantageous to provide two valves in said steam inlet line and to branch said condensate drain line between these two valves. For sterilization steam is introduced through the first valve and the second valve into the equipment. The shut off valve in the condensate drain line will close as described above. During normal operation the first and the second valve are closed. When a leakage in the first valve occurs steam will leave the system via the condensate drain line. The second valve still blocks the entry of any steam into the equipment.

To ensure that in case both valves leak no steam flows into the equipment it is preferred to operate the equipment in normal operation at a higher pressure than the pressure of the steam at the steam inlet. Thus, if both valves leak steam cannot enter the equipment due to the pressure difference between the steam side of the second valve and the equipment side of the second valve. In that case the fluid flowing in the equipment will flow through the second valve out of the equipment and into the steam inlet line and prevent steam from entering the equipment.

In normal operation the equipment is preferably used for-the production of a sterile cryogenic liquid, in particular for the production of sterile liquid nitrogen, sterile liquid oxygen, sterile liquid argon or sterile liquid carbon dioxide.

That production step preferably comprises the steps of sterilizing a gas by passage through a filter unit and condensing said sterilized gas in a condenser to achieve a sterile cryogenic liquid. In order to guarantee the required purity of the sterilized cryogenic liquid product it is advantageous to have alternately a sterilization step and a production step.

A preferred mode of operation would be as follows: First the whole equipment is sterilized by introduction of clean steam as described above. The steri lization is carried out for a sufficient time at a sufficient high temperature to ensure that all parts of the equipment are completely sterilized. Then any remaining water is drained from the equipment and a gas is introduced in order to dry the equipment. Preferably said drying gas is heated prior to entering the equipment. When the drying step is finished the introduced gas is used to cool down the equipment and thus the gas is no more heated prior to entering the equipment. When the equipment is cooled down the production can start. The introduced gas is passed through the filter unit and sterilized. The sterilized gas is then liquified in the condenser by indirect heat exchange with a cryogenic coolant and withdrawn as sterile cryogenic liquid product.

The gas used for drying and cooling the equipment is the same gas which shall later be produced. For example, when producing sterile liquid nitrogen the equipment will be dried and cooled with gaseous nitrogen. The cryogenic coolant is preferably liquid nitrogen.

In case sterile liquid nitrogen shall be produced the cryogenic coolant is also liquid nitrogen, but it is preferably taken from another source than the nitrogen gas to be sterilized. For use as cryogenic coolant liquid nitrogen of lower quality and hence lower cost can be used. When the customer has high quality and purity demands on the sterile cryogenic liquid, it is necessary to introduce already relative pure nitrogen gas of high quality into the equipment. In that case it is a big benefit to have different sources of nitrogen, one of lower quality to be used as cryogenic coolant, one of high quality which will then be sterilized. So the operation costs can be reduced essentially.

To achieve as high efficiency as possible it is advantageous to introduce gaseous nitrogen or any other gas that shall be sterilized into the equipment at a pressure of at least 5 bar, more preferred at least 8 bar. The pressure in the equipment is preferably controlled by a control valve located downstream the condenser which is used for liquifying the sterile gas. That control valve is operated to control the discharge of produced sterile cryogenic liquid. In order to achieve full sterilization of that control valve it is advantageous to have steam flowing through the housing of the control valve during the sterilization step.

During sterilization the pressure of the clean steam should preferably be about between 2 and 4 bar. On the one hand, such a steam pressure is high enough to ensure that the steam flows into all parts of the equipment. On the other hand, the steam pressure is below the pressure within the equipment at normal operation. This is a benefit since it ensures that no steam can enter the equipment even if there is a leak in the valve(s) in the steam inlet line.

As mentioned above it is advantageous to have all parts of the equipment drainable. For that reason the product withdrawal pipeline downstream the condenser should also preferably be provided with a drain line. However, during normal operation sterile cryogenic liquid flows within this pipeline. Thus a drain valve located in the drain line has to be aseptic, but aseptic shut off valves are not commercially available for operating at cryogenic temperatures. If a cryogenic liquid enters such a valve there is a considerable risk of failure.

Thus, according to a preferred embodiment of the invention a main pipeline or any other part of the equipment containing a cryogenic liquid is provided with a drain line comprising a drain valve wherein said drain line between said main pipeline and said drain valve is heated. For example, the drain line branching from the main pipeline comprises a spiral tube with a rather small inner diameter. This spiral tube is continuously heated with an electric heater, for example an electrical tracing cable. In this way, any cryogenic liquid flowing downwards through the drain line is evaporated before reaching the drain valve. The evaporated cryogenic liquid builds a protecting gas buffer between the main pipeline containing cryogenic liquid and the drain valve. It is then possible to use an aseptic drain valve in a drain line connected to a main pipeline containing cryogenic liquids.

During the sterilization step the drain valve is open and steam and condensate can easily pass through the spiral tube, or in general through the drain line, and the drain valve. The drain line or especially the spiral tube are designed self-draining so that there is no water left after the sterilization.

The invention as well as further details and preferred embodiments are disclosed in the following description and illustrated in the accompanying drawings, in which the figures show different modes of operation of the invention, namely
- figure 1: the production mode,
- figure 2: the heating mode,
- figure 3: the sterilization mode, and
- figure 4: the drying mode.

The figures schematically show an inventive equipment for the production of sterile liquid nitrogen. Of course, with some obvious adaptions the equipment may also be , used for the production of any other sterile cryogenic liquid.

The inventive equipment comprises a storage tank 1 for liquid nitrogen coolant. Storage tank 1 is connected via pipe 2 with valve 3 to condenser 4. Downstream condenser 4 a valve 5 is provided in order to control the flow of liquid nitrogen through condenser 4.

Another gaseous nitrogen source 6, for example the head space of a liquid nitrogen tank, is connected via line 7 to heat exchanger 8. Downstream heat exchanger 8 gaseous nitrogen from nitrogen source 6 flows through line 9 with valve 10 to a sterile filter 11. Sterile gaseous nitrogen leaving the sterile filter 11 flows through line 17 to condenser 4 where it is condensed in indirect heat exchange with the liquid nitrogen coolant. The discharge amount of sterile liquid nitrogen produced in condenser 4 can be controlled by sterile valve 12. The produced sterile liquid nitrogen is withdrawn through pipeline 13 which can be closed by an aseptic valve 14. A drain line 15 provided with an aseptic valve 16 branches upstream valve 14. Drain line 15 is completely drainable, that is it slopes down all the way without any low points. Valve 16 is an aseptic valve. Such aseptic valves are designed for ambient temperatures or at least for temperatures above -40°C, but there are no aseptic valves available that withstand cryogenic temperatures.

In order to prevent cryogenic liquid to flow down to aseptic valve 16 drain line 15 is provided with a spiral tube 34 of small inner diameter. An electric heater 35 mounted close to spiral tube 34 continuously heats up spiral tube 34. Thus, any cryogenic liquid flowing down through spiral tube 34 is vaporized. The resulting vapour creates a gas buffer above aseptic valve 16 protecting said valve 16.

Line 17 between sterile filter 11 and condenser 4 further comprises a branch line 18 with valve 19 which directly connects line 17 with the stem housing of sterile valve 12. To ensure sterility in the extended housing of sterile valve 12, clean steam will be introduced into the top of the stem housing during the sterilization mode as will be explained later. In addition line 17 is provided with a drain line 20 with drain valve 21.

Condenser 4 is designed as a double walled heat exchanger to minimize the risk of cross contamination in case of leakage. That means that there is a space ventilated to the atmosphere between the two pressurized sides of condenser 4. If there is a leakage in any of the passages, it will leak into the ambient atmosphere instead of into the clean passages where the sterile nitrogen flows.

In heat exchanger 8 gaseous nitrogen can be heated in indirect heat exchange with steam. For that reason plant steam can be fed through line 22 to heat exchanger 8. The amount of plant steam can be controlled by a steam trap 40. The steam leaving heat exchanger 8 is withdrawn via line 24.

For sterilization purposes a source 25 of clean steam is connected to line 9. The main problem in having a steam inlet is that it is not allowed to have any leakages of steam into the sterile nitrogen production. There are two main reasons for this: First, any water in the nitrogen passages might clog condenser 4 or any other part of the equipment when it freezes. Second, the quality requirements for the sterile nitrogen allow only a maximum content of water, for example 5 ppm, which must not be exceeded.

To avoid these problems steam inlet 26 comprises two valves 27, 28 and a drain line 36 branching between the two valves 27, 28. Drain line 36 is provided with a steam trap 29 which acts a shut off function. Below a certain switch temperature steam trap 29 is open, above that switch temperature it closes.

When the equipment is in production mode both valves 27, 28 are closed and steam cannot enter line 9. If a leakage in valve 27 appears the steam will be drained through the steam trap 29 which is open since it has not been heated by steam and thus the temperature is below the switch temperature.

If there is also a leakage in valve 28 the higher pressure in the gaseous nitrogen supply lines 7, 9 will force gaseous nitrogen through valve 28 and line 28 into steam trap 29. Thus no steam will reach the sterile parts of the equipment. For that reason the pressure in the gaseous nitrogen supply 6 has to be above the pressure of the clean steam 25. In practice the gaseous nitrogen pressure is preferably between 7 and 10 bars whereas the clean steam pressure is preferably between 2 and 4 bars.

During the sterilization mode both valves 27, 28 are open and hot steam is allowed to flow through line 26. The hot steam will also enter drain line 36 and heat up steam trap 29 which will close as soon as the switch temperature is reached.

For sterilization purposes sterile filter 11 is also provided with a line 30 interconnecting the bottom and the top of the housing of filter 11. Line 30 is provided with two valves 31, 32 and a drain line 33.

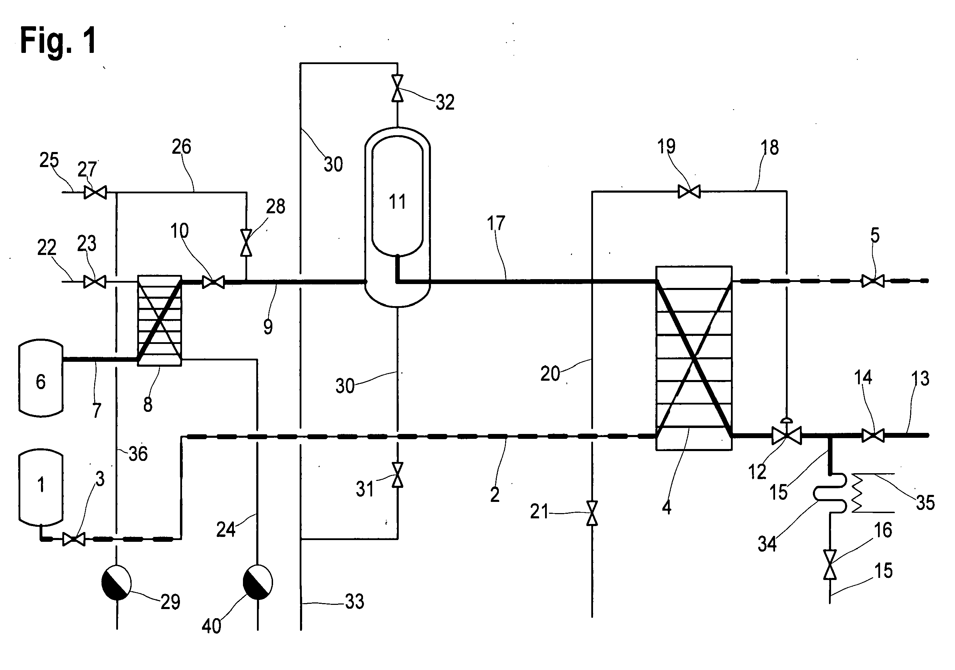

The function of the equipment shall now be explained with reference to figures 1 to 4 wherein same reference numbers refer to same parts. The equipment can be run in different modes of operation:
- Stop mode
- Production of sterile liquid nitrogen
- Heating from cryogenic temperatures to ambient temperature
- Sterilization
- Drying and cooling down

In the stop mode all valves 3, 5, 12, 14, 16, 19, 21, 23, 27, 28, 31, 32 are closed except valve 10 which is opened to keep a pressure in the equipment. Valve 5 is designed to be opened manually to release the pressure on the cooling side if necessary.

Figure 1 shows the equipment in the production mode. The flow path of gaseous nitrogen and sterile nitrogen is shown in bold lines, the flow path of the liquid nitrogen coolant in dashed line. Gaseous nitrogen is withdrawn from nitrogen tank 6 and fed through heat exchanger 8 to sterile filter 11. Valve 23 is closed. Hence heat exchanger 8 is not provided with plant steam since there is no need to heat up the gaseous nitrogen.

In filter 11 the gaseous nitrogen is sterilized and then transferred as sterile gaseous nitrogen to condenser 4. In condenser 4 the sterile gaseous nitrogen is condensed in indirect heat exchange with liquid nitrogen coolant withdrawn from liquid nitrogen storage tank 1.

In order to achieve as high efficiency as possible the pressure of the gaseous nitrogen should be at least 8 bars. This is done by controlling the discharge amount of produced sterile liquid nitrogen downstream condenser 4 by valve 12. The condensation temperature is controlled by controlling the flow of liquid nitrogen coolant using valve 5. On/off valve 3 is only used to block the flow of liquid nitrogen coolant if the equipment stops the production.

From time to time the whole equipment has to be sterilized which is done by passing clean steam through the equipment. In order to avoid clogging of passages or pipelines by freezing water the equipment has first to be heated to about ambient temperature.

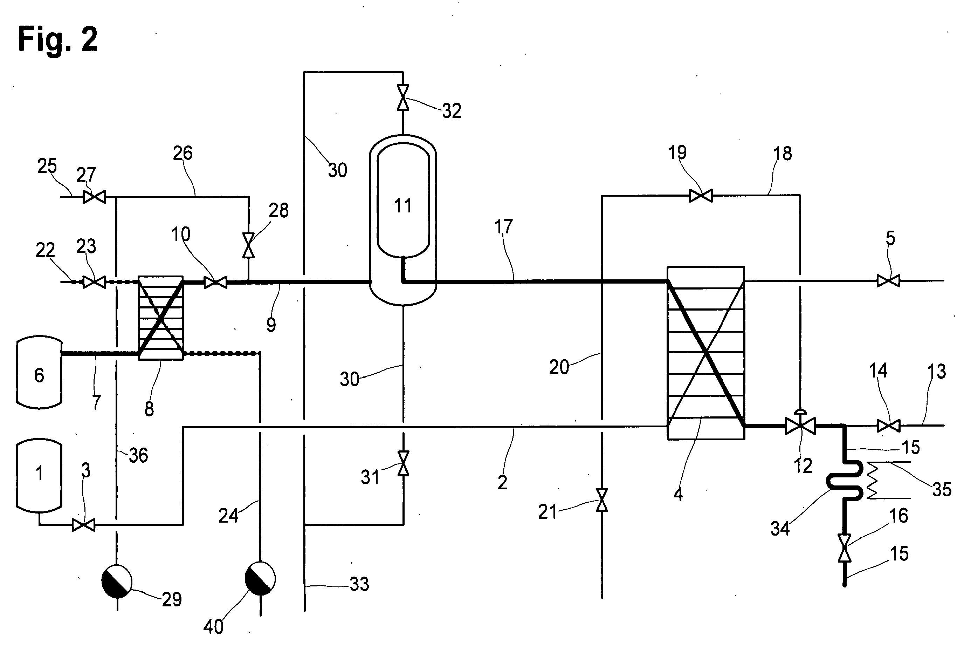

The heating mode is shown in figure 2. The flow path of gaseous nitrogen is again shown in bold lines, the flow path of the plant steam in dotted line.

Valve 3 is closed in order to stop the flow of liquid nitrogen coolant to condenser 4. The heating is performed by a heated gaseous nitrogen flow. Valves 10, 12, 16 are opened and a flow of gaseous nitrogen from nitrogen source 6, through heat exchanger 8, sterile filter 11 and condenser 4 to drain line 15 is generated. This gaseous nitrogen flow is heated in heat exchanger 8 in indirect heat exchange with steam. The heating of the equipment is continued until a temperature of 20 °C is reached. Then valves 10 and 23 are closed to interrupt the flow of gaseous nitrogen and the flow of plant steam.

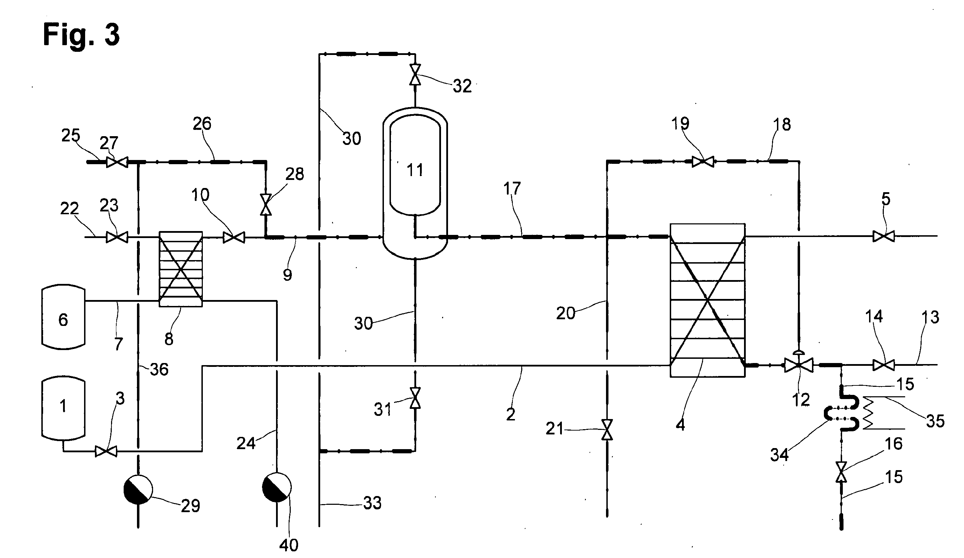

When the heating is finished the sterilization mode starts as shown in figure 3. Sterilization is achieved by passing clean steam into the equipment. Opening of valves 27, 28, 12, 16, 19, 21, 31, 32 generate a flow of clean steam (shown in dashed-dotted lines). The clean steam flow will continue until all parts of the equipment have reached a temperature of 125°C. This is controlled by several temperature controllers not shown in the drawing. When the whole equipment has reached the temperature of 125°C the clean steam flow will continue for another 15 minutes. During this time none of the temperatures controlled by the temperature controllers is allowed to drop below 121 °C in order to guarantee sufficient sterilization. If the temperature falls below 121 °C the sterilization mode has to be started once again. During the sterilization period valve 12 is not always completely open, but changes its opening according to a pre-defined pattern.

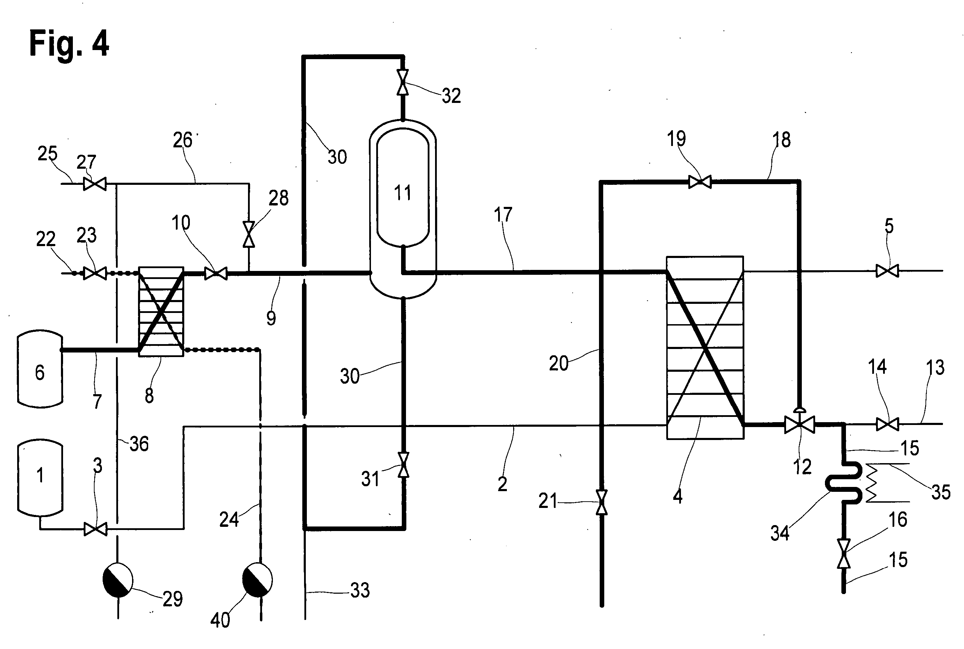

When the sterilization is finished the equipment has to be dried in order to prevent the equipment from freezing when the production starts. The respective flow paths are shown in figure 4 wherein the flow path of gaseous nitrogen is shown in bold lines and the flow path of plant steam in dotted line.

The drying is performed by a heated gaseous nitrogen stream. Gaseous nitrogen is withdrawn from nitrogen source 6 and passed to heat exchanger 8 where it is heated in indirect heat exchange with hot plant steam. The heated nitrogen gas is passed through sterile filter 11, condenser 4 and stem housing of valve 12. In a first step the entire equipment is dried. After a pre-set time valves 31 and 32 are closed and the drying of the remaining parts is continued for a pre-set time.

After the drying step valve 23 is closed to stop the flow of steam to heat exchanger 8. The gaseous nitrogen from source 6 is no more heated and will cool down the filter 11 and the subsequent parts of the equipment. The cool down mode is dependent on the size of the equipment. Then the cooling is finished and all valves except valve 10 will be closed. The whole system is now ready for starting a new production mode. Valve 10 is left open to keep a minimum pressure in the equipment while it is not in operation.

## Claims

1. Method for sterilizing in place of equipment (9, 11,17, 4,12) wherein in operation said equipment (9, 11,17, 4,12) is used at cryogenic temperatures comprising the steps of
- passing steam through said equipment (9, 11,17, 4,12) and
- drying said equipment (9, 11,17, 4,12) by introducing a gas into said equipment (9, 11,17, 4,12),
**characterized in that**
- prior to passing steam through said equipment (9, 11,17, 4,12), said equipment (9, 11,17, 4,12) is pre-heated by introduction of a gas.

2. Method according to claim 1 wherein said equipment (9, 11,17, 4,12) is pre-heated and / or dried by the same type of gas which said equipment (9, 11,17, 4,12) contains at normal operation.

3. Method according to any of claims 1 or 2 wherein said equipment (9, 11,17, 4,12) is designed to be completely drainable.

4. Method according to any of claims 1 to 3 wherein the geometrical design of said equipment (9, 11,17, 4,12) is in such a way that all parts of said equipment (9, 11,17, 4,12) are reached by said steam.

5. Method according to any of claims 1 to 4 wherein said steam is introduced into said equipment (9, 11,17, 4,12) by a steam inlet line (25, 26) comprising at least one valve (27, 28) and a drain line (36) branching upstream said valve (27, 28) wherein said drain line (36) is provided with a shut off valve (29).

6. Method according to any of claims 1 to 5 wherein said equipment (9, 11,17, 4,12) is provided with a drain line (15, 34) comprising a drain valve (16) wherein said drain line (15, 34) is heated.

7. Method according to any of claims 1 to 6, wherein in operation said equipment (9, 11,17, 4,12) is used for the production of a sterile cryogenic liquid, in particular for the production of sterile liquid nitrogen.

8. Method according to claim 7 wherein said equipment comprises a filter unit (11) and a condenser (4) and wherein said production of said sterile cryogenic liquid comprises the steps of:
- sterilizing a gas by passage through said filter unit (11) and
- condensing said sterilized gas in said condenser (4) to achieve a sterile cryogenic liquid.

9. Method according to.claim 8 wherein said sterilized gas is condensed in indirect heat exchange with a cryogenic coolant wherein said gas to be sterilized and said cryogenic coolant are taken from different sources.

10. Method according to any of claims 7 to 9 wherein the pressure of said gas to be sterilized is greater than the pressure of said cryogenic coolant.

11. Method according to any of claims 7 to 10 wherein the pressure of said gas to be sterilized is greater than 5 bar.
